# EUROPÄISCHE PATENTANMELDUNG

(11) **EP 0 572 893 A1**
(43) Veröffentlichungstag der Anmeldung: **08.12.1993**
(21) Anmeldenummer: 93108357.0
(22) Anmeldetag: 24.05.1993
(51) Int. Cl.: C07D 263/58, A01N 43/76

(54) **Fluorbenzoxazolyloxyacetamide**

(30) Priorität: 04.06.1992 DE 4218433
(71) Anmelder: BAYER AG, 51368 Leverkusen (DE)
(72) Erfinder: Förster, Heinz, Dr., W-5600 Wuppertal (DE); Böhm, Stefan, Dr., W-5000 Köln 80 (DE); Marhold, Albrecht, Dr., W-5090 Leverkusen (DE); Santel, Hans-Joachim, Dr., W-5090 Leverkusen (DE); Lürssen, Klaus, dr., W-5060 Bergisch Gladbach 2 (DE); Schmidt, Robert R., Dr., W-5060 Bergisch Gladbach 2 (DE)

(57) **Zusammenfassung**

Die Erfindung betrifft neue Fluorbenzoxazolyloxyacetamide der Formel (I) 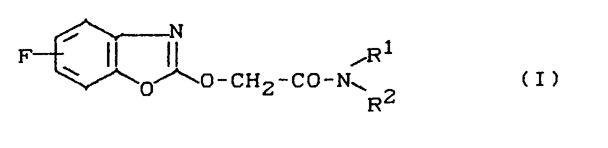
in welcher
- R¹: für Wasserstoff oder für einen gegebenenfalls substituierten Rest aus der Reihe Alkyl, Alkenyl, Alkinyl oder Aralkyl steht,
- R²: für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder
- R¹ und R²: zusammen mit dem Stickstoffatom, an welches sie gebunden sind, einen gegebenenfalls substituierten, gesättigten oder ungesättigten Stickstoff-Heterocyclus bilden, der weitere Heteroatome enthalten kann und an den eine Benzo-Gruppierung anelliert sein kann,
ein Verfahren zu ihrer Herstellung und ihre Verwendung als Herbizide.

## Beschreibung

Die Erfindung betrifft neue Fluorbenzoxazolyloxyacetamide, ein Verfahren und neue Zwischenprodukte zu ihrer Herstellung sowie ihre Verwendung als Herbizide.

Es ist bereits bekannt, daß bestimmte Benzazolyloxyacetamide, wie z.B. Benzthiazolylessigsäure-N-methyl-anilid, herbizide Eigenschaften aufweisen (vgl. EP-A 5501; US-A-4.509.971 und US-A-4.833.243). Vorbekannt sind auch verschiedene, gegebenenfalls substituierte Benzoxazolyloxyacetamide mit herbiziden Eigenschaften (vgl. z.B. EP-A-29 171, US-A-4.408.055; EP-A-161 602, US-A-4.784.682; DE-A 3 038 652; DE-A-4 133 673; ferner WO 91/06544). Die herbizide Wirksamkeit dieser Verbindungen ist jedoch nicht immer ganz zufriedenstellend.

Es wurden nun neue Fluorbenzoxazolyloxyacetamide der allgemeinen Formel (I) gefunden, 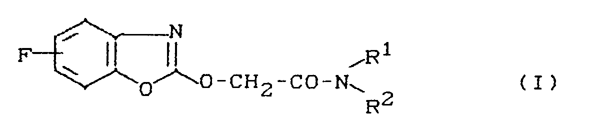
in welcher
- R¹: für Wasserstoff oder für einen gegebenenfalls substituierten Rest aus der Reihe Alkyl, Alkenyl, Alkinyl oder Aralkyl steht,
- R²: für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder
- R¹ und R²: zusammen mit dem Stickstoffatom, an welches sie gebunden sind, einen gegebenenfalls substituierten, gesättigten oder ungesättigten Stickstoff-Heterocyclus bilden, der weitere Heteroatome enthalten kann und an den eine Benzo-Gruppierung anelliert sein kann.

Weiter wurde gefunden, daß man die neuen Fluorbenzoxazolyloxyacetamide der Formel (I) erhält, wenn man 2-Chlorfluorbenzoxazole der allgemeinen Formel (II) 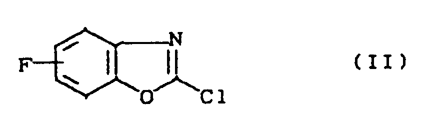
mit Hydroxyacetamiden der allgemeinen Formel (III) 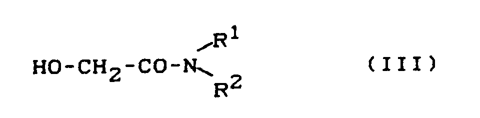
in welcher
- R¹ und R²: die oben angegebenen Bedeutungen haben,
gegebenenfalls in Gegenwart eines Verdünnungsmittels, gegebenenfalls in Gegenwart eines Säurebindemittels und gegebenenfalls in Gegenwart eines Katalysators umsetzt.

Schließlich wurde gefunden, daß die neuen Fluorbenzoxazolyloxyacetamide der allgemeinen Formel (I) interessante herbizide Eigenschaften besitzen.

Überraschenderweise zeigen die erfindungsgemäßen Verbindungen der Formel (I) bei teilweise sehr guter Verträglichkeit gegenüber Kulturpflanzen, wie z.B. Reis, erheblich stärkere Wirkung gegen schwer bekämpfbare Unkräuter, wie z.B. Hühnerhirse (Echinochloa crus galli), als die chemisch ähnliche bekannte Verbindung Benzthiazolyloxyessigsäure-N-methyl-anilid.

Gegenstand der Erfindung sind vorzugsweise Verbindungen der Formel (I), in welcher
- R¹: für Wasserstoff, C₁-C₈-Alkyl, welches gegebenenfalls durch Fluor, Chlor, Cyano oder C₁-C₄-Alkoxy substituiert ist, für C₂-C₈-Alkenyl, welches gegebenenfalls durch Fluor und/oder Chlor substituiert ist, für C₂-C₈-Alkinyl oder für Benzyl steht,
- R²: für C₁-C₈-Alkyl, welches gegebenenfalls durch Fluor, Chlor, Cyano oder C₁-C₄-Alkoxy substituiert ist, für C₂-C₈-Alkenyl, welches gegebenenfalls durch Fluor und/oder Chlor substituiert ist, für C₂-C₈-Alkinyl, für C₃-C₆-Cycloalkyl, welches gegebenenfalls durch Chlor und/oder C₁-C₃-Alkyl substituiert ist, für C₅- oder C₆-Cycloalkenyl, für Benzyl, welches gegebenenfalls durch Fluor, Chlor und/oder C₁-C₄-Alkyl substituiert ist, für Phenyl, welches gegebenenfalls durch Fluor, Chlor, Brom, Iod, Cyano, Nitro, C₁-C₄-Alkyl, Trifluormethyl, C₁-C₄-Alkoxy und/oder C₁-C₄-Alkylthio substituiert ist, für C₁-C₈-Alkoxy, welches gegebenenfalls durch C₁-C₄-Alkoxy substituiert ist, oder für C₃-C₄-Alkenyloxy steht, oder
- R¹ und R²: zusammen mit dem Stickstoffatom, an das sie gebunden sind, einen gegebenenfalls ein- bis dreifach durch C₁-C₃-Alkyl substituierten, gesättigten oder ungesättigten fünf- bis siebengliedrigen Stickstoffheterocyclus bilden, welcher gegebenenfalls benzanelliert ist.

Die Erfindung betrifft insbesondere Verbindungen der Formel (I), in welcher
- R¹: für C₁-C₄-Alkyl, Allyl oder Propargyl steht,
- R²: für C₁-C₆-Alkyl, C₁-C₂-Alkoxy-C₁-C₂-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy oder Ethoxy substituiert ist), C₁-C₆-Alkoxy oder C₁-C₂-Alkoxy-C₁-C₂-alkoxy steht, oder
- R¹ und R²: zusammen mit dem Stickstoffatom, an das sie gebunden sind, für gegebenenfalls ein- bis dreifach durch Methyl und/oder Ethyl substituiertes Piperidinyl, für gegebenenfalls ein- oder zweifach durch Methyl und/oder Ethyl substituiertes Pyrrolidinyl, für Perhydroazepinyl oder für 1,2,3,4-Tetrahydro(iso)-chinolinyl stehen.

Ganz besonders bevorzugte Gruppen von Verbindungen der Formel (I) werden nachstehend durch die Formeln (IA), (IB) und (IC) wiedergegeben, wobei R¹ und R² die oben als bevorzugt bzw. als insbesondere bevorzugt angegebenen Bedeutungen haben. 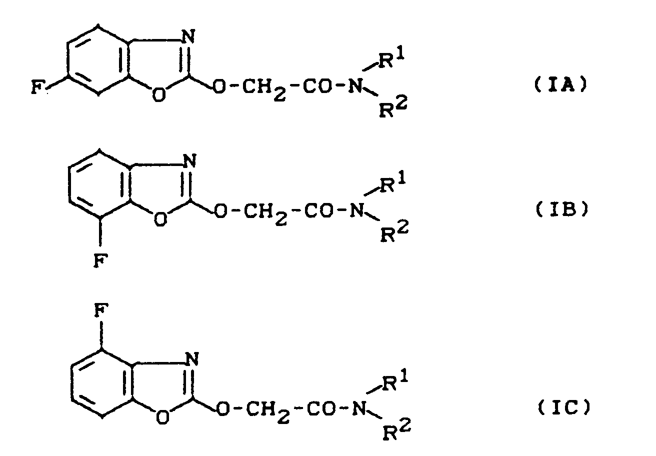
Die oben aufgeführten allgemeinen oder in Vorzugsbereichen aufgeführten Restedefinitionen bzw. Erläuterungen gelten für die Endprodukte und für die Ausgangs- und Zwischenprodukte entsprechend. Diese Restedefinitionen können untereinander, also auch zwischen den jeweiligen Vorzugsbereichen beliebig kombiniert werden.

Beispiele für die möglichen Bedeutungen
der Gruppierung 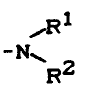
in den Formeln (I), (IA), (IB) und (IC) sind in der nachstehenden Tabelle 1 aufgeführt.

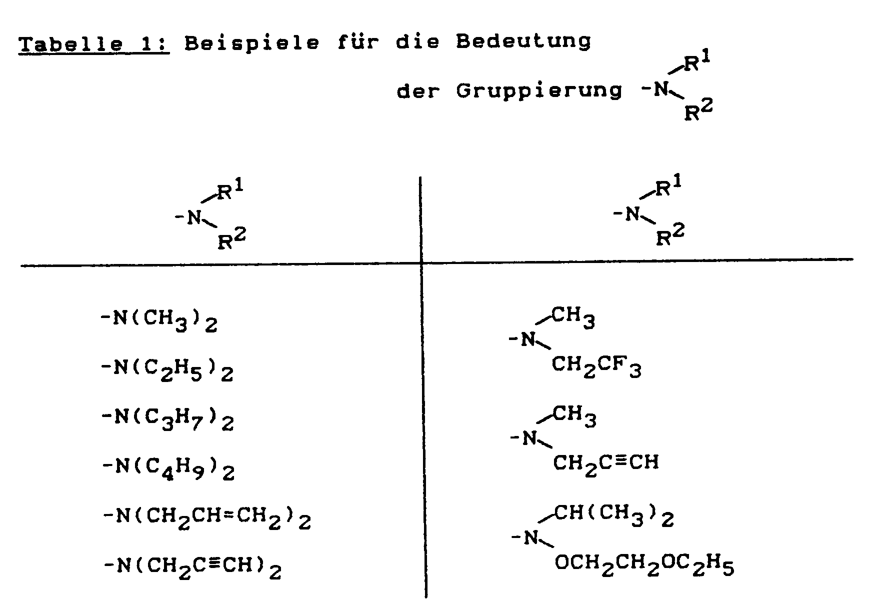

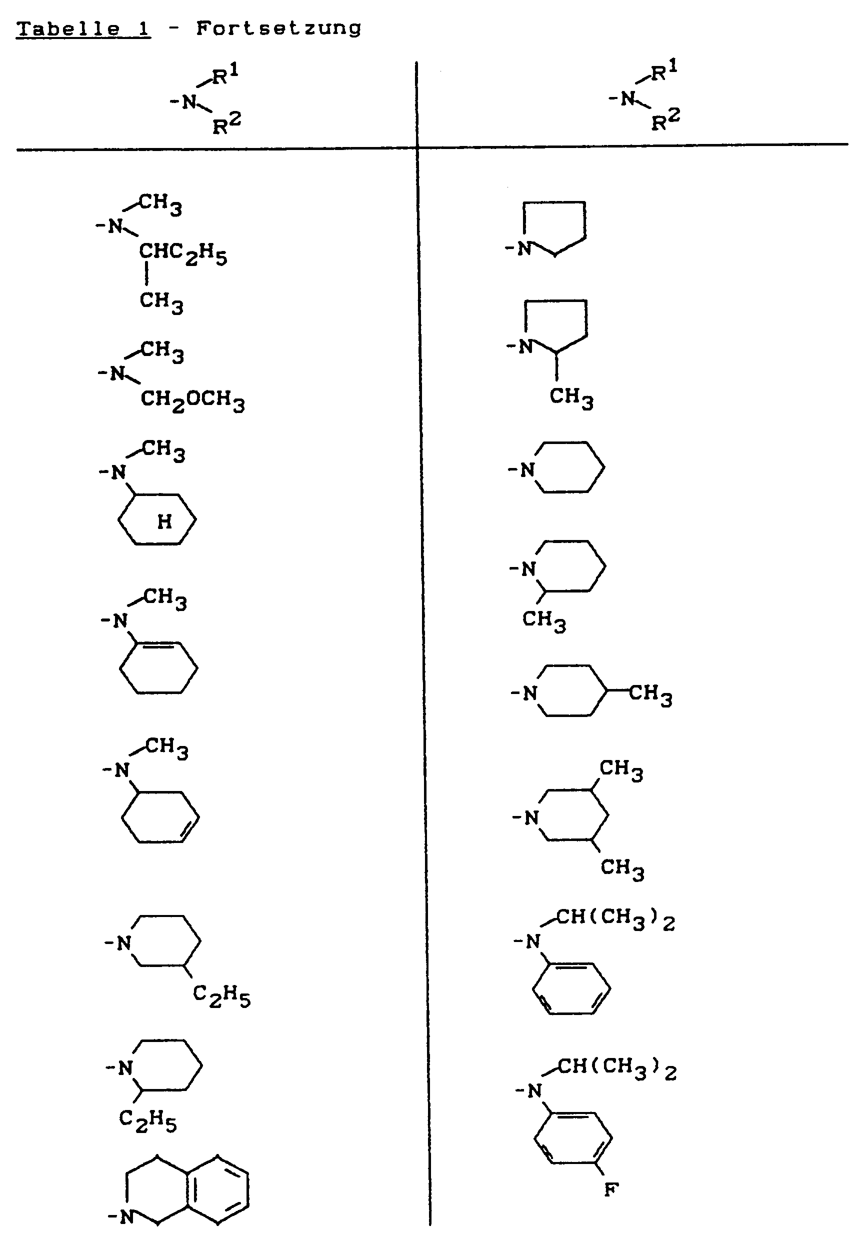

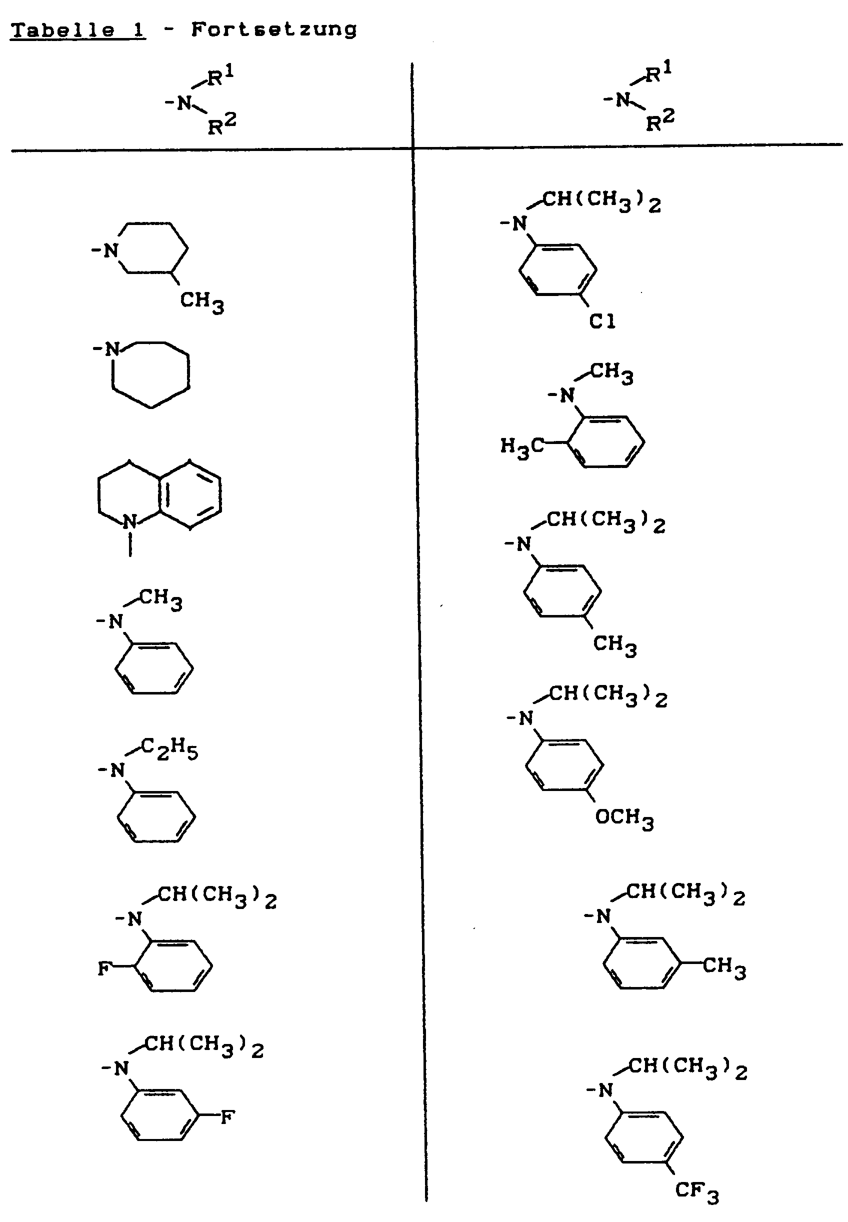

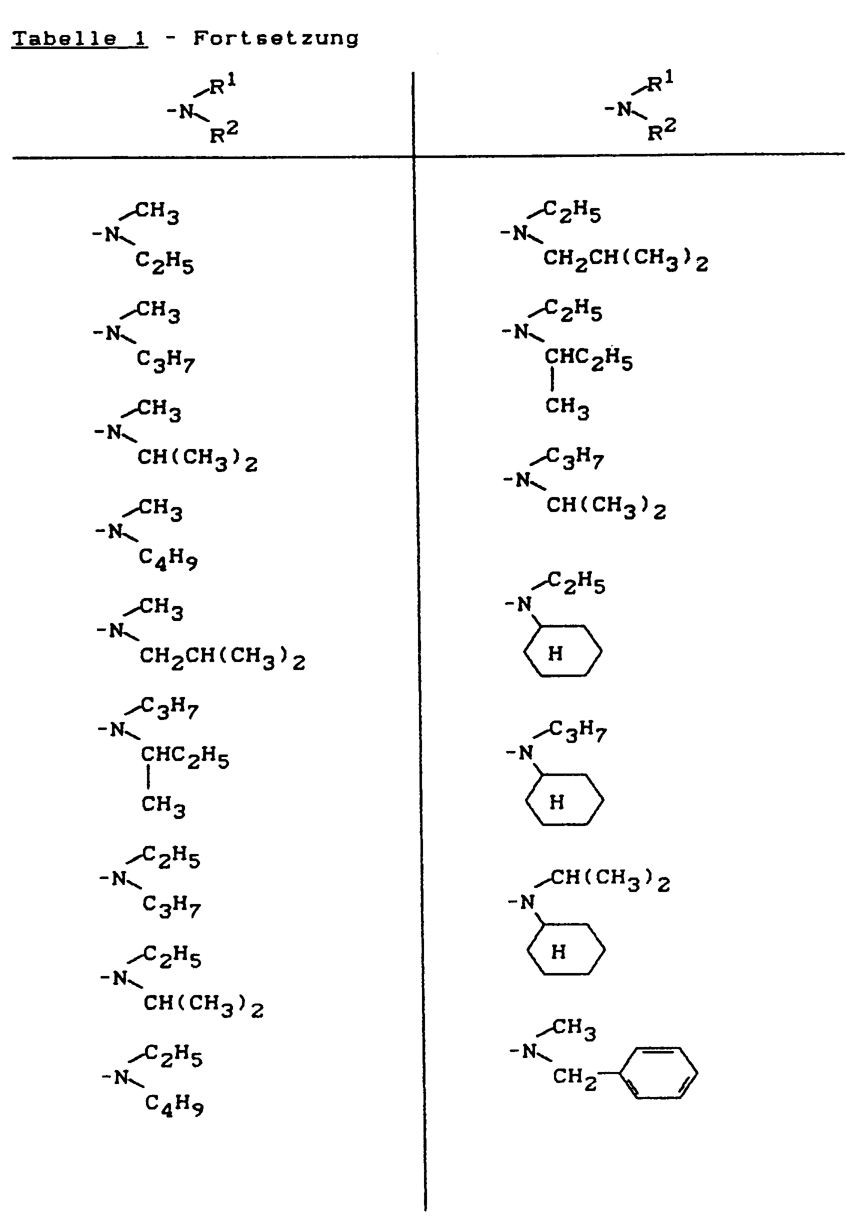

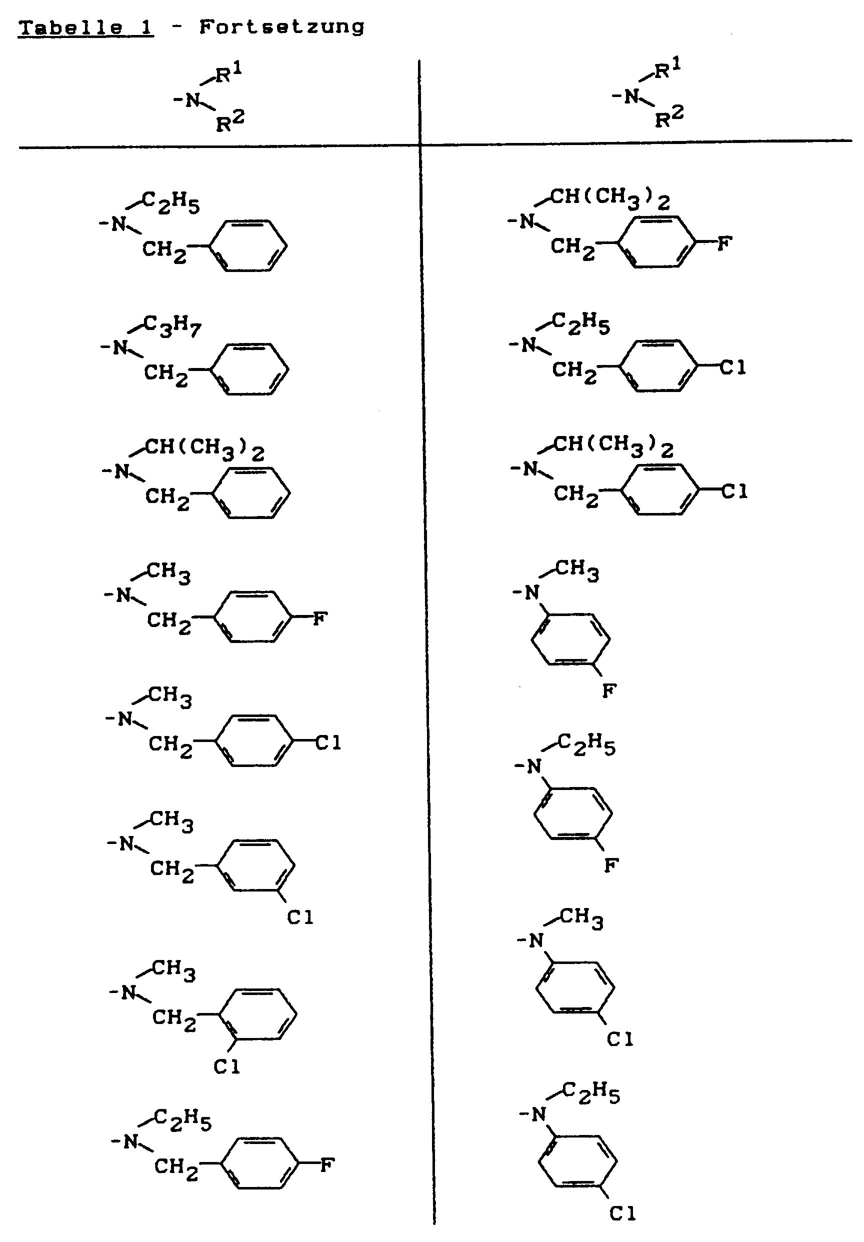

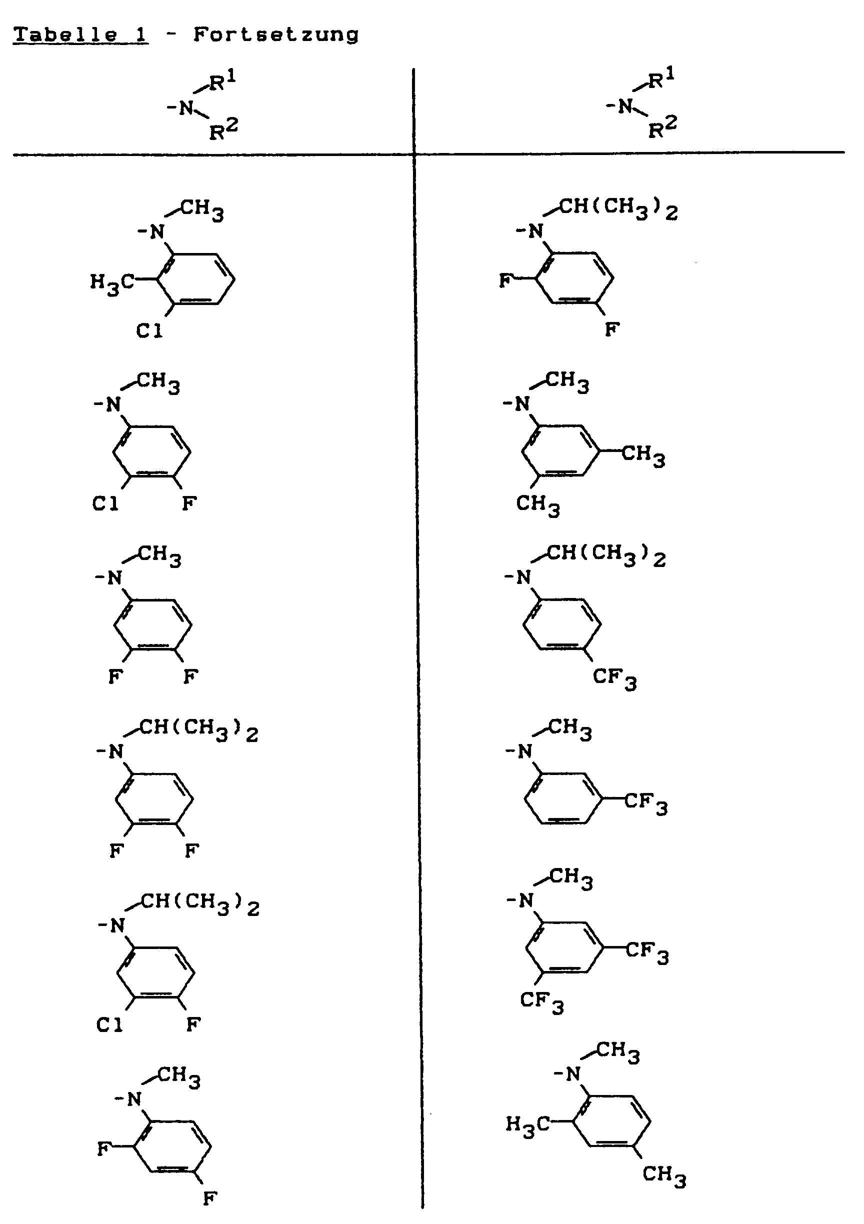

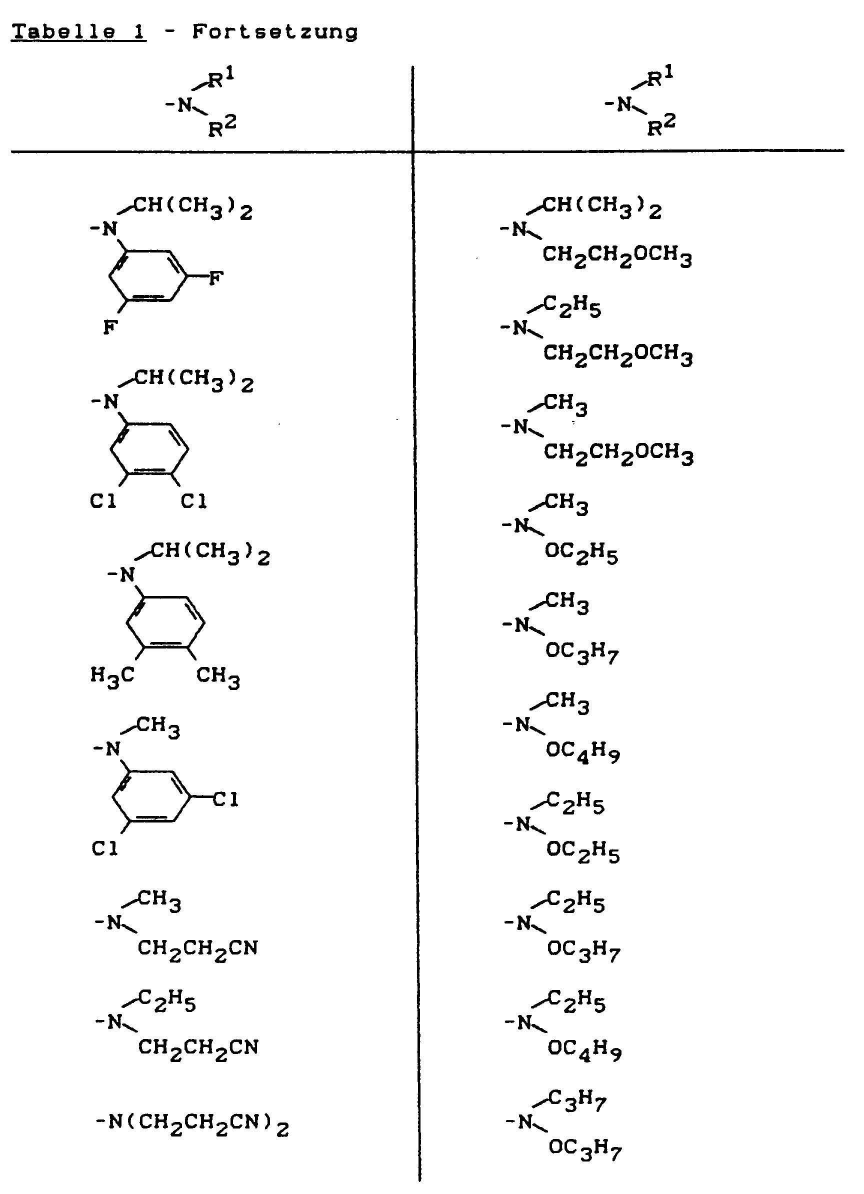

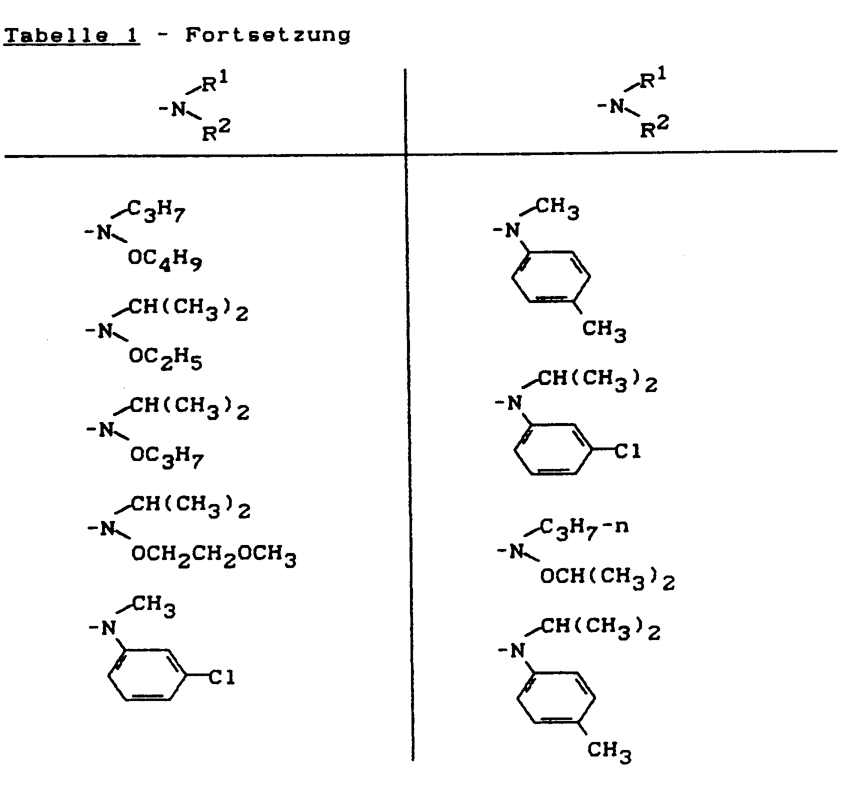

Verwendet man beispielsweise 2-Chlor-6-fluor-benzoxazol und N,N-Diethyl-hydroxyacetamid als Ausgangsstoffe, so läßt sich der Reaktionsablauf beim erfindungsgemäßen Herstellungsverfahren durch das folgende Formelschema darstellen: 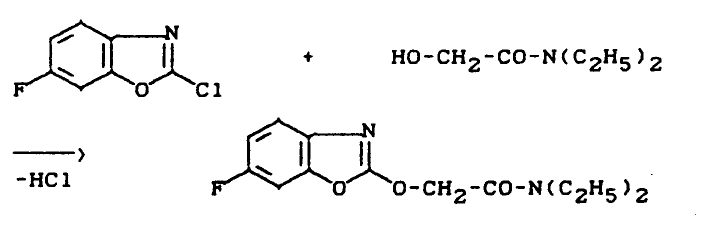
Die beim erfindungsgemäßen Herstellungsverfahren für die Verbindungen der Formel (I) als Ausgangsstoffe zu verwendenden 2-Chlorfluorbenzoxazole sind mit Ausnahme von 2-Chlor-5-fluor-benzoxazol (vgl. WO 9106544 - zitiert in Chem. Abstracts 115:92253d) noch nicht aus der Literatur bekannt.

Neu und als neue Stoffe ebenfalls Gegenstand der vorliegenden Erfindung sind also 2-Chlor-4-fluor-benzoxazol, 2-Chlor-6-fluor-benzoxazol und 2-Chlor-7-fluor-benzoxazol.

Man erhält die 2-Chlorfluorbenzoxazole der allgemeinen Formel (II), wenn man 2-Mercaptofluorbenzoxazole der allgemeinen Formel (IV) 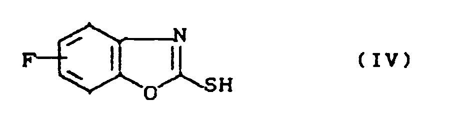
mit Chlorierungsmitteln, wie z.B. Thionylchlorid oder Phosphorylchlorid, gegebenenfalls in Gegenwart eines Reaktionshilfsmittels, wie z.B. Dimethylformamid, und gegebenenfalls in Gegenwart eines Verdünnungsmittels, wie z.B. Chloroform oder Tetrachlormethan, bei Temperaturen zwischen 0°C und 100°C umsetzt (vgl. die Herstellungsbeispiele).

Die als Vorprodukte benötigten 2-Mercaptofluorbenzoxazole sind mit Ausnahme von 5-Fluor-2-mercapto-benzoxazol (vgl. US-P 4476137; J. Med. Chem. 10 (1967), 408-410) noch nicht aus der Literatur bekannt.

Als neue Stoffe gleichfalls Gegenstand der vorliegenden Erfindung sind also 4-Fluor-2-mercapto-benzoxazol, 6-Fluor-2-mercapto-benzoxazol und 7-Fluor-2-mercapto-benzoxazol.

Man erhält die 2-Mercaptofluorbenzoxazole der allgemeinen Formel (IV), wenn man fluorierte 2-Aminophenole der allgemeinen Formel (V) 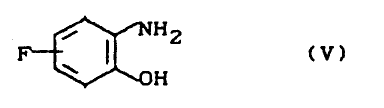
mit Xanthogenaten der allgemeinen Formel (VI) 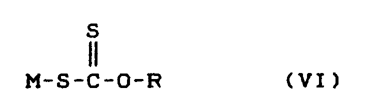
in welcher
- M: für ein Metall, vorzugsweise Natrium oder Kalium, steht und
- R: für Alkyl, vorzugsweise Methyl oder Ethyl, steht,
in Gegenwart von Verdünnungsmitteln, wie z.B. Wasser, Methanol und/oder Ethanol, bei Temperaturen zwischen 0°C und 120°C umsetzt (vgl. die Herstellungsbeispiele).

Die als Vorprodukte benötigten fluorierten 2-Aminophenole der Formel (V) sind bekannt und/oder können nach an sich bekannten Verfahren hergestellt werden (vgl. EP-A 415641; EP-A 415642; EP 385664; US-P 4144235; Bull. Chem. Soc. Japan 47 (1974), 2079-2080; J. Am. Chem. Soc. 81 (1959), 94).

Die weiter als Vorprodukte benötigten Xanthogenate der Formel (VI) sind bekannte, handelsübliche Synthesechemikalien.

Die beim erfindungsgemäßen Verfahren zur Herstellung von Verbindungen der Formel (I) weiter als Ausgangsstoffe zu verwendenden Hydroxyessigsäureamide sind durch die Formel (III) allgemein definiert.

In Formel (III) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden.

Die Hydroxyessigsäureamide der Formel (III) sind bekannt und/oder können nach an sich bekannten Verfahren hergestellt werden (vgl. US-P 4 509 971 und US-P 4 645 525; ferner US-P 4 334 073, DE-OS 3 038 598, DE-OS 3 038 636, EP-A 37 526, EP-A 348 737, DE-OS 38 19 477).

Das erfindungsgemäße Verfahren zur Herstellung der neuen Fluorbenzoxazolyloxyacetamide der Formel (I) wird vorzugsweise unter Verwendung von Verdünnungsmitteln duchgeführt. Hierzu gehören vorzugsweise Kohlenwasserstoffe, wie z. B. Toluol, Xylol oder Cyclohexan, Halogenkohlenwasserstoffe, wie z. B. Methylenchlorid, Ethylenchlorid, Chloroform oder Chlorbenzol, Ether, wie z. B. Diethylether, Dipropylether, Diisopropylether, Dibutylether, Diisobutylether, Glycoldimethylether, Tetrahydrofuran und Dioxan, Alkohole, wie z. B. Methanol, Ethanol, Propanol, Isopropanol, Butanol, Isobutanol, sec-Butanol oder tert-Butanol, Ketone, wie z. B. Aceton, Methylethylketon, Methylisopropylketon und Methylisobutylketon, Ester, wie z. B. Essigsäuremethylester und Essigsäureethylester, Amide, wie z. B. Dimethylformamid, Dimethylacetamid und N-Methyl-pyrrolidon, Nitrile, wie z. B. Acetonitril und Propionitril, Sulfoxide, wie z. B. Dimethylsulfoxid sowie Wasser oder wäßrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt ist Natriumchlorid,

Das erfindungsgemäße Verfahren wird vorteilhaft unter Verwendung von Säurebindemitteln durchgeführt. Als solche werden vorzugsweise stark basische Alkali- und Erdalkalimetallverbindungen, beispielsweise Oxide, wie z.B. Natrium-, Kalium-, Magnesium- und Calciumoxid, Hydroxide, wie z. B. Natrium-, Kalium-, Magnesium- und Calciumhydroxid, Alkoholate, wie z.B. Natrium- und Kalium-tertbutylat und/oder Carbonate, wie z. B. Natrium-, Kalium-, Magnesium- und Calclumcarbonat verwendet.

Der Zusatz von 0,01 bis 10 Gew.-% (bezogen auf eingesetztes Glycolsäureamid der Formel (III)) eines Phasentransferkatalysators mag sich in einigen Fällen als vorteilhaft erweisen. Als Beispiele für solche Katalysatoren seien genannt:
Tetrabutylammoniumchlorid, Tetrabutylammoniumbromid, Tributyl-methylphosphoniumbromid, Trimethyl-C₁₃/C₁₅-alkyl-ammoniumchlorid, Dibenzyl-dimethyl-ammonium-methylsulfat, Dimethyl-C₁₂/C₁₄-alkyl-benzylammoniumchlorid, Tetrabutylammoniumhydroxid, 18-Krone-6, Triethylbenzylammoniumchlorid, Trimethylbenzylammoniumchlorid, Tetraethylammoniumbromid.

Die Reaktionstemperaturen können bei dem erfindungsgemäßen Verfahren in einem größeren Bereich variiert werden. Im allgemeinen arbeitet man bei Temperaturen zwischen -50 °C und +110 °C, vorzugsweise bei Temperaturen zwischen -20 °C und +80 °C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemäßen Verfahrens setzt man je Mol Chlorfluorbenzoxazol der Formel (II) im allgemeinen 0,5 bis 5 Mol, vorzugsweise 0,8 bis 1,5 Mol, Hydroxyessigsäureamid der Formel (III) ein. Die Reaktionskomponenten können in beliebiger Reihenfolge zusammengegeben werden. Man rührt jeweils das Reaktionsgemisch bis zum Ende der Umsetzung und arbeitet nach üblichen Methoden auf (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen Unkräutern in verpflanztem Wasserreis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Losungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

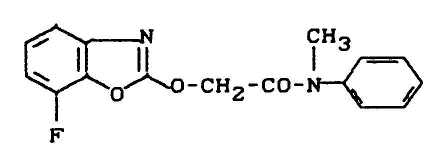

Eine Mischung aus 2,4 g (15 mMol) N-Methyl-N-phenyl-hydroxyacetamid, 1,7 g (15 mMol) Kallum-tert-butylat, 11 ml Acetonitril und 30 ml tert-Butanol wird auf 0°C abgekühlt und bei 0°C bis +5°C wird eine Lösung von 2,6 g (15 mMol) 2-Chlor-7-fluor-benzoxazol in 4 ml Acetonitril tropfenweise dazugegeben. Das Reaktionsgemisch wird 12 Stunden bei 0°C bis +5°C gerührt, dann mit Wasser auf etwa das doppelte Volumen verdünnt und das kristallin angefallene Produkt durch Absaugen isoliert.

Man erhält 3,3 g (73% der Theorie) N-Methyl-N-phenyl-α-(7-fluor-benzoxazol-2-yl-oxy)-acetamid vom Schmelzpunkt 150°C.

Analog Beispiel 1 und entsprechend der allgemeinen Beschreibung des erfindungsgemäßen Herstellungsverfahrens können beispielsweise auch die in der nachstehenden Tabelle 2 aufgeführten Verbindungen der Formel (I) hergestellt werden.

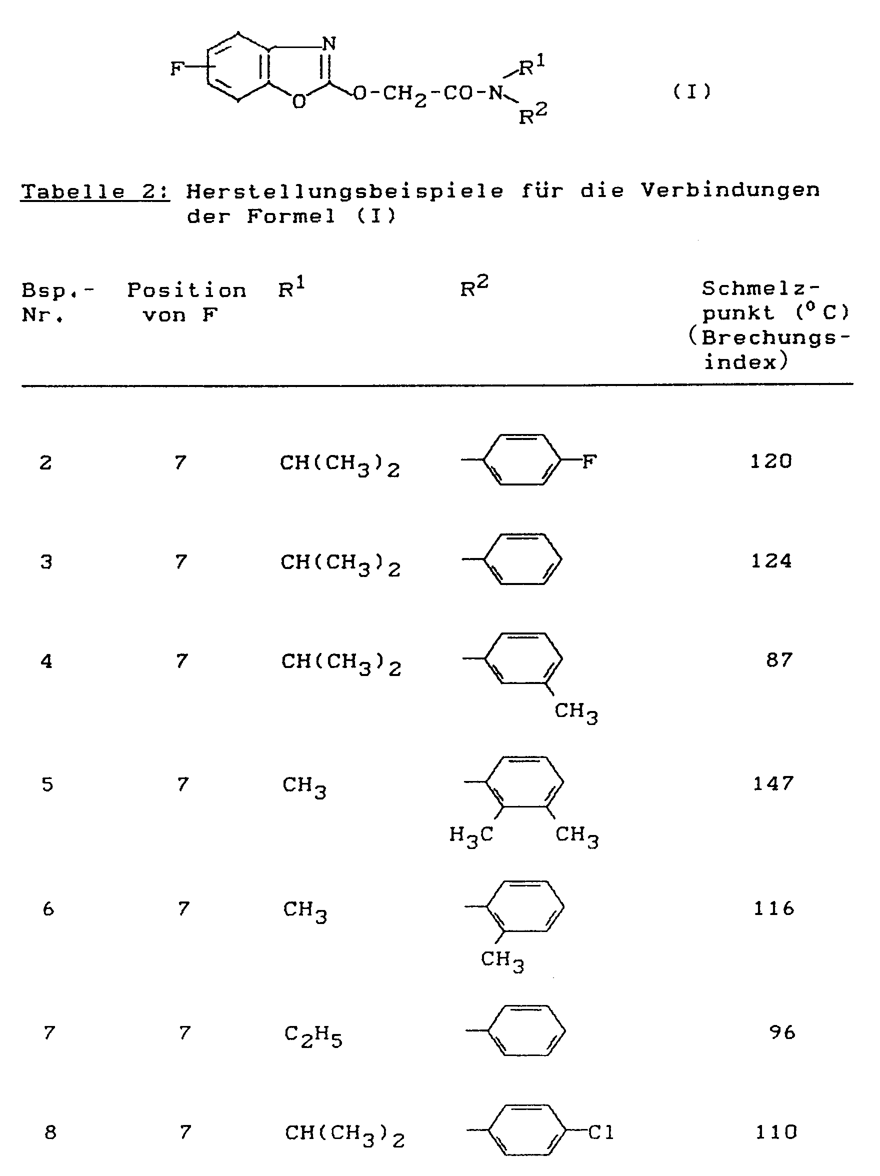

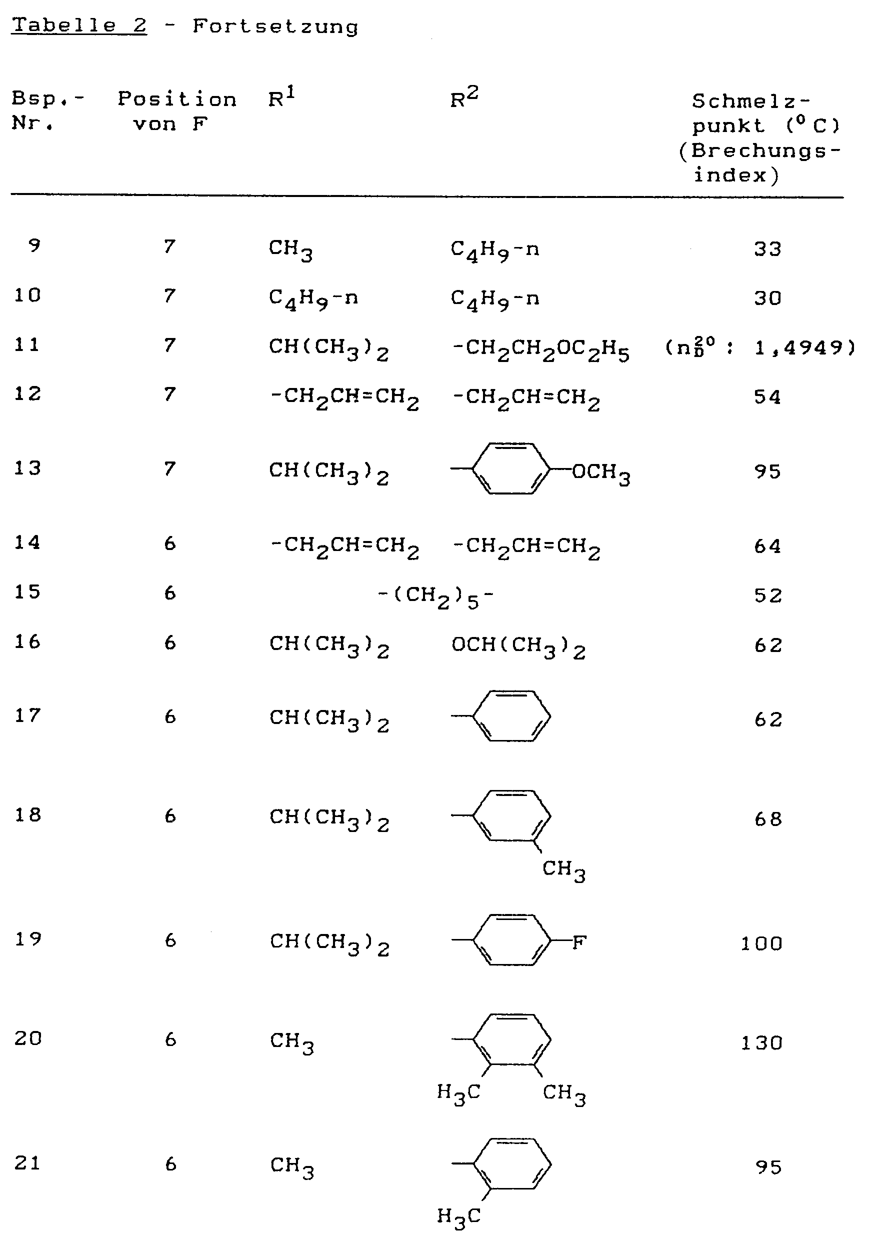

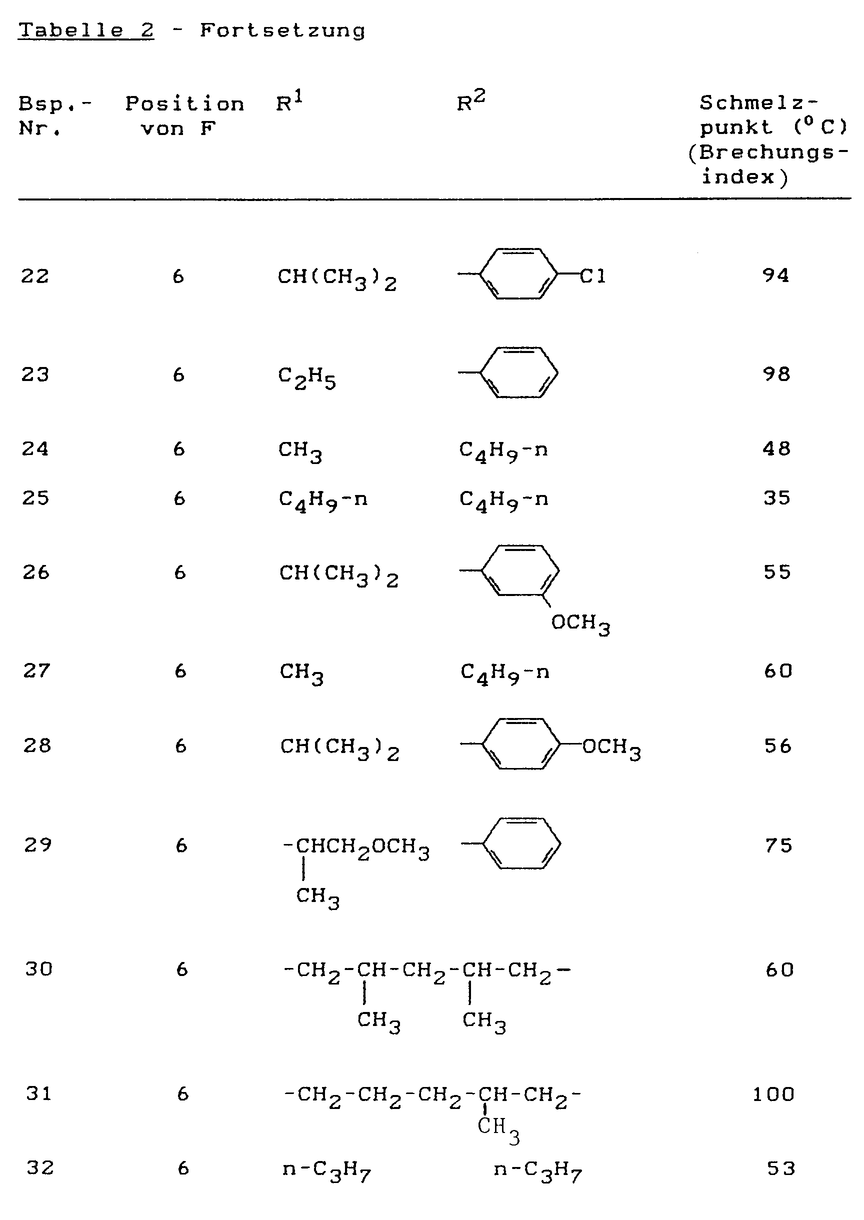

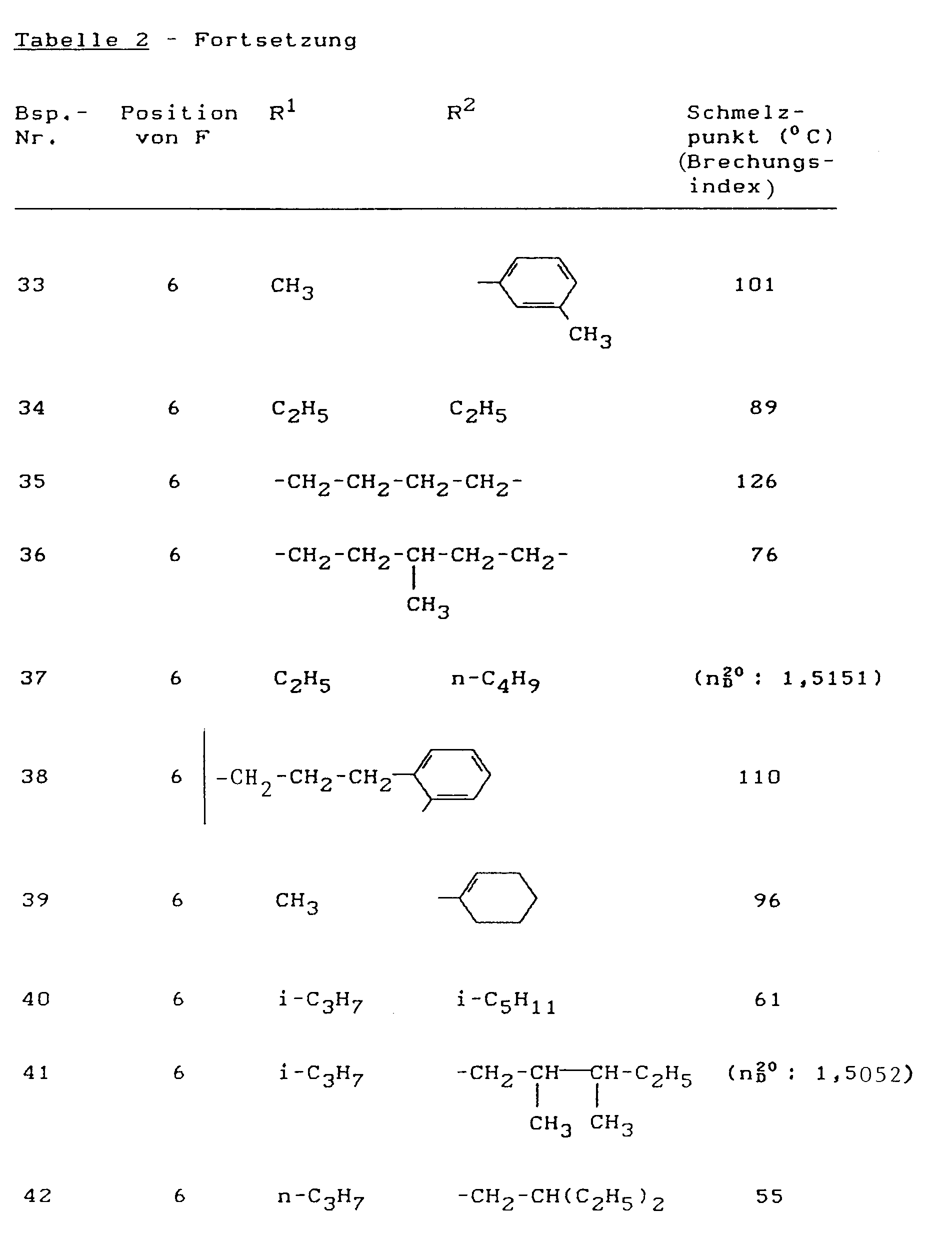

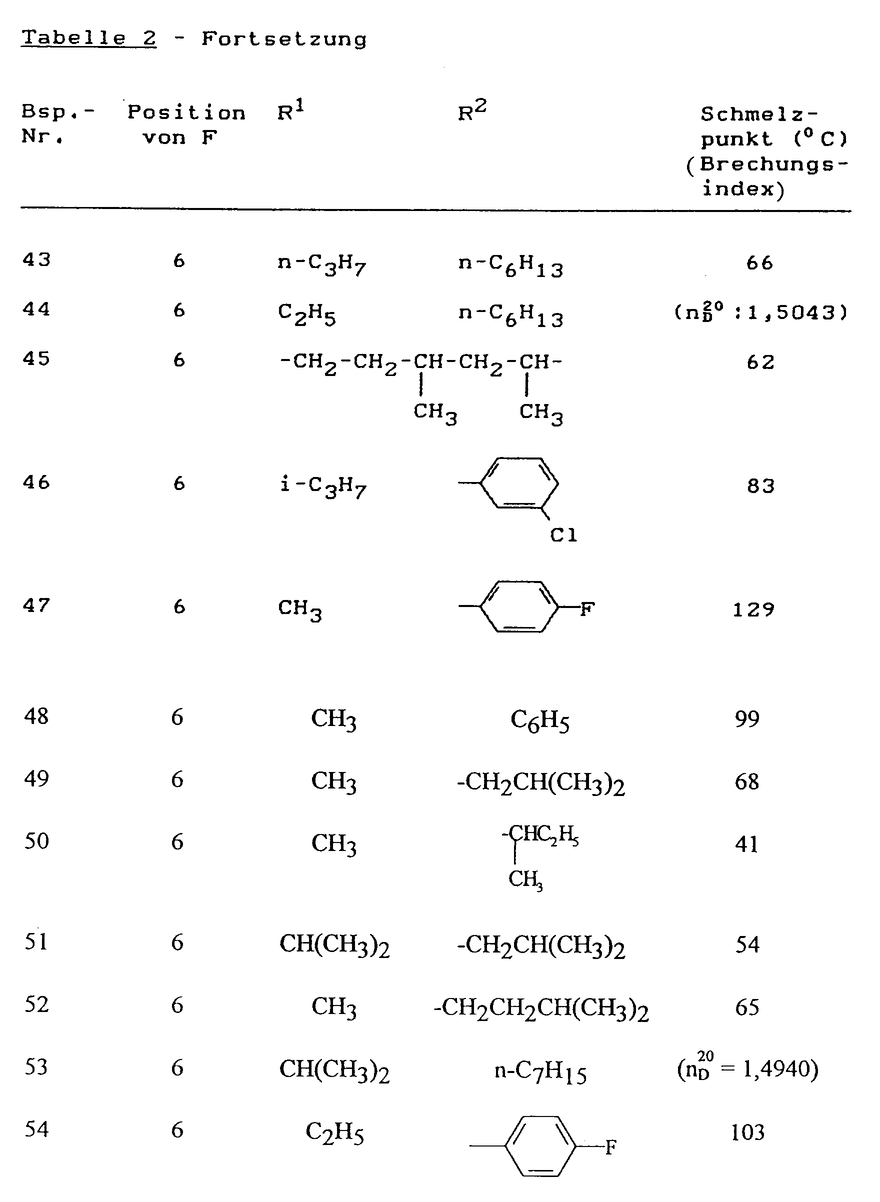

### Ausgangsstoffe der Formel (II):

### Beispiel (II-1)

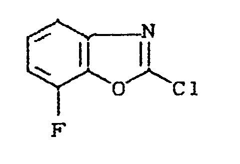

60 ml Thionylchlorid werden in kleinen Portionen zu einer Mischung aus 56 g (0,33 Mol) 7-Fluor-2-mercapto-benzoxazol und 12 ml Dimethylformamid gegeben und das Gemisch wird dann auf 70°C erwärmt. Dann werden weitere 123 ml Thionylchlorid innerhalb einer Stunde so zugetropft, daß die Innentemperatur 70°C nicht übersteigt. Das komplette Reaktionsgemisch wird 2 Stunden unter Rückfluß erhitzt und dann unter vermindertem Druck eingeengt. Der Rückstand wird in 800 ml Cyclohexan aufgenommen und mit Aktivkohle geklärt. Nach Einengen im Wasserstrahlvakuum wird das im Rückstand verbliebene Produkt durch Destillation im Ölpumpenvakuum gereinigt.

Man erhält 25 g (45% der Theorie) 2-Chlor-7-fluor-benzoxazol vom Siedepunkt 50°C bei 0,4 mbar.

### Beispiel (II-2)

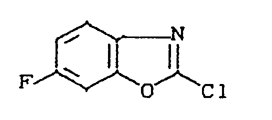

100 ml Thionylchlorid werden in kleinen Portionen zu einer Mischung aus 102 g (0,60 Mol) 6-Fluor-2-mercapto-benzoxazol und 18 ml Dimethylformamid gegeben und das Gemisch wird dann auf 70°C erwärmt. Dann werden weitere 300 ml Thionylchlorid innerhalb einer Stunde so zugetropft, daß die Innentemperatur 70°C nicht übersteigt. Das komplette Reaktionsgemisch wird dann 2 Stunden unter Rückfluß erhitzt und dann unter vermindertem Druck eingeengt. Der Rückstand wird in 500 ml Cyclohexan aufgenommen und mit Aktivkohle geklärt. Nach Einengen im Wasserstrahlvakuum wird das im Rückstand verbliebene Produkt durch Destillation im Ölpumpenvakuum gereinigt.

Man erhält 83 g (81% der Theorie) 2-Chlor-6-fluor-benzoxazol vom Siedepunkt 55°C bei 1 mbar.

### Ausgangsstoffe der Formel (IV):

### Beispiel (IV-1)

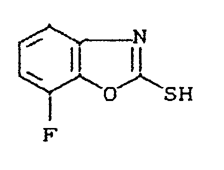

Eine Mischung aus 48 g (0,38 Mol) 2-Amino-6-fluor-phenol, 64 g (0,40 Mol) Kalium-ethylxanthogenat und 200 ml Wasser wird 3 Stunden unter Rückfluß erhitzt. Anschließend werden 600 ml Wasser und 100 ml konzentrierte Salzsäure dazugegeben und das kristallin angefallene Produkt wird durch Absaugen isoliert.

Man erhält 56,7 g (89% der Theorie) 7-Fluor-2-mercapto-benzoxazol vom Schmelzpunkt 216°C.

### Beispiel (IV-2)

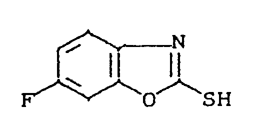

Eine Lösung von 302 g (1,88 Mol) Kalium-ethylxanthogenat in 200 ml Wasser wird zu einer Mischung aus 218 g (1,71 Mol) 2-Amino-5-fluor-phenol und 1,7 l Ethanol gegeben und das komplette Gemisch wird 4,5 Stunden unter Rückfluß erhitzt. Anschließend wird der Alkohol weitgehend abdestilliert, der Rest mit 4 l Wasser versetzt und mit Essigsäure angesäuert. Das kristallin angefallene Produkt wird durch Absaugen isoliert.

Man erhält 261 g (100% der Theorie) 6-Fluor-2-mercapto-benzoxazol vom Schmelzpunkt 245°C.

### Anwendungsbeispiele:

In den nachfolgenden Anwendungsbeispielen wird die nachstehend aufgeführte Verbindung als Vergleichssubstanz herangezogen: 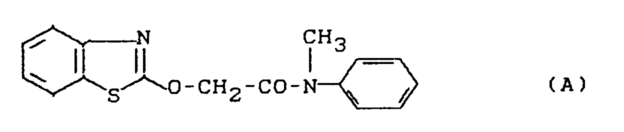
Benzthiazol-2-yl-oxy-essigsäure-N-methyl-anilid (bekannt aus EP-A 5501, Bsp. 1)

### Beispiel A: Pre-emergence-Wasseroberflächenbehandlung bei verpflanztem Wasserreis

Zur Herstellung einer applizierfähigen Zubereitung wird 1 Teil Wirkstoff mit 5 Teilen Aceton gelöst; anschließend wird 1 Teil Benzyloxy-polyglykol-ether als Emulgator zugegeben. Dann wird Wasser bis zur gewünschten Konzentration zugegeben. In Töpfe, die mit Erde gefüllt sind, wird Reis im 2-3-Blattstadium verpflanzt. Samen von Testpflanzen werden ausgelegt (1 cm tief). Zwei Tage später werden die Töpfe mit Wasser 3 cm überstaut. Anschließend werden die Wirkstoffzubereitungen auf die Wasseroberfläche ausgebracht. 4 Wochen später wird die herbizide Wirkung und die Schädigung der behandelten Pflanzen im Vergleich zu unbehandelten visuell in % ausgewertet. 0% bedeutet keine Wirkung, 100% bedeutet völliges Absterben.

In diesem Test zeigt sich die zum Teil sehr gute Verträglichkeit der erfindungsgemäßen Wirkstoffe - insbesondere der Verbindungen aus den Herstellungsbeispielen 1, 2, 3, 4, 5, 6, 7, 8, 9, 10, 11, 12 und 13 - in Reis bei im Vergleich mit der bekannten Verbindung (A) wesentlich stärkerer Wirkung gegen Unkräuter, insbesondere gegen Hühnerhirse (Echinochloa crus galli).

## Patentansprüche

1. Fluorbenzoxazolyloxyacetamide der allgemeinen Formel (I), 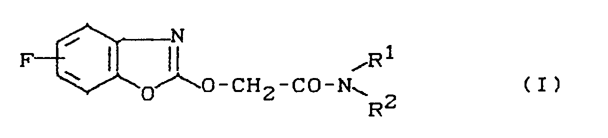 in welcher
R¹ für Wasserstoff oder für einen gegebenenfalls substituierten Rest aus der Reihe Alkyl, Alkenyl, Alkinyl oder Aralkyl steht,
R² für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder
R¹ und R² zusammen mit dem Stickstoffatom, an welches sie gebunden sind, einen gegebenenfalls substituierten, gesättigten oder ungesättigten Stickstoff-Heterocyclus bilden, der weitere Heteroatome enthalten kann und an den eine Benzo-Gruppierung anelliert sein kann.

2. Fluorbenzoxazolyloxyacetamide der allgemeinen Formel (I) gemäß Anspruch 1, dadurch gekennzeichnet, daß darin
R¹ für Wasserstoff, C₁-C₈-Alkyl, welches gegebenenfalls durch Fluor, Chlor, Cyano oder C₁-C₄-Alkoxy substituiert ist, für C₂-C₈-Alkenyl, welches gegebenenfalls durch Fluor und/oder Chlor substituiert ist, für C₂-C₈-Alkinyl oder für Benzyl steht,
R² für C₁-C₈-Alkyl, welches gegebenenfalls durch Fluor, Chlor, Cyano oder C₁-C₄-Alkoxy substituiert ist, für C₂-C₈-Alkenyl, welches gegebenenfalls durch Fluor und/oder Chlor substituiert ist, für C₂-C₈-Alkinyl, für C₃-C₆-Cycloalkyl, welches gegebenenfalls durch Chlor und/oder C₁-C₃-Alkyl substituiert ist, für C₅- oder C₆-Cycloalkenyl, für Benzyl, welches gegebenenfalls durch Fluor, Chlor und/oder C₁-C₄-Alkyl substituiert ist, für Phenyl, welches gegebenenfalls durch Fluor, Chlor, Brom, Iod, Cyano, Nitro, C₁-C₄-Alkyl, Trifluormethyl, C₁-C₄-Alkoxy und/oder C₁-C₄-Alkylthio substituiert ist, für C₁-C₈-Alkoxy, welches gegebenenfalls durch C₁-C₄-Alkoxy substituiert ist, oder für C₃-C₄-Alkenyloxy steht, oder
R¹ und R² zusammen mit dem Stickstoffatom, an das sie gebunden sind, einen gegebenenfalls ein-bis dreifach durch C₁-C₃-Alkyl substituierten, gesättigten oder ungesättigten fünf- bis siebengliedrigen Stickstoffheterocyclus bilden, welcher gegebenenfalls benzanelliert ist.

3. Fluorbenzoxazolyloxyacetamide der allgemeinen Formel (I) gemäß Anspruch 1, dadurch gekennzeichnet, daß darin
R¹ für C₁-C₄-Alkyl, Allyl oder Propargyl steht,
R² für C₁-C₆-Alkyl, C₁-C₂-Alkoxy-C₁-C₂-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy oder Ethoxy substituiert ist), C₁-C₆-Alkoxy oder C₁-C₂-Alkoxy-C₁-C₂-alkoxy steht, oder
R¹ und R² zusammen mit dem Stickstoffatom, an das sie gebunden sind, für gegebenenfalls ein- bis dreifach durch Methyl und/oder Ethyl substituiertes Piperidinyl, für gegebenenfalls ein-oder zweifach durch Methyl und/oder Ethyl substituiertes Pyrrolidinyl, für Perhydroazepinyl oder für 1,2,3,4-Tetrahydro(iso)-chinolinyl stehen.

4. 4-Fluor-, 6-Fluor- und 7-Fluor-benzoxazolyloxy-acetamide der Formeln (IC, (IA) bzw. (IB) gemäß Ansprüchen 1-3:

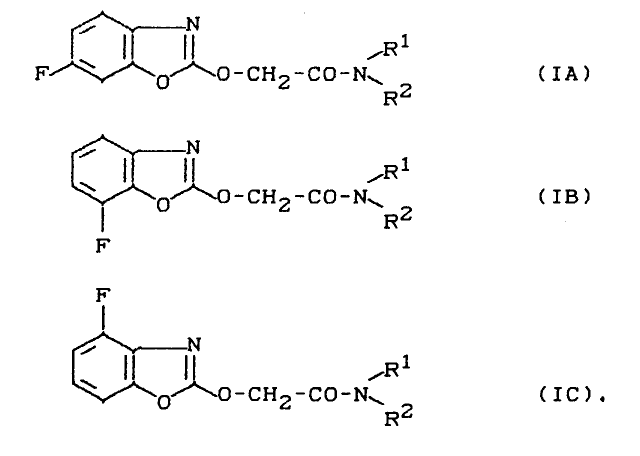

5. Verfahren zur Herstellung von Fluorbenzoxazolyloxyacetamiden der Formel (I) gemäß Anspruch 1, dadurch gekennzeichnet, daß man 2-Chlor-fluorbenzoxazole der Formel (II) 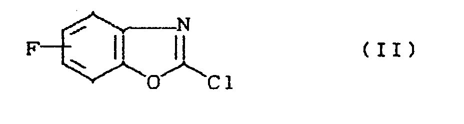 mit Hydroxyacetamiden der allgemeinen Formel (III) 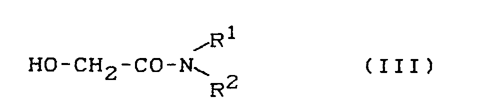 in welcher
R¹ und R² die in Anspruch 1 angegebenen Bedeutungen haben,
gegebenenfalls in Gegenwart eines Verdünnungsmittels, gegebenenfalls in Gegenwart eines Säurebindemittels und gegebenenfalls in Gegenwart eines Katalysators umsetzt.

6. Herbizide Mittel, gekennzeichnet durch einen Gehalt an mindestens einer Verbindung der Formel (I) gemäß Anspruch 1.

7. Verwendung von Verbindungen der allgemeinen Formel (I) gemäß Anspruch 1 zur Bekämpfung von unerwünschtem Pflanzenwachstum.

8. Verfahren zur Bekämpfung von Unkräutern, dadurch gekennzeichnet, daß man Verbindungen der allgemeinen Formel (I) gemäß Anspruch 1 auf die Unkräuter oder ihren Lebensraum einwirken läßt.

9. Verfahren zur Herstellung von herbiziden Mitteln, dadurch gekennzeichnet, daß man Verbindungen der allgemeinen Formel (I) gemäß Anspruch 1 mit Streckmitteln und/oder oberflächenaktiven Mitteln vermischt.

10. Die Verbindungen
2-Chlor-4-fluor-benzoxazol,
2-Chlor-6-fluor-benzoxazol,
2-Chlor-7-fluor-benzoxazol,
4-Fluor-2-mercapto-benzoxazol,
6-Fluor-2-mercapto-benzoxazol und
7-Fluor-2-mercapto-benzoxazol.
